# DEMANDE DE BREVET EUROPEEN

(11) **EP 4 275 695 A1**
(43) Date de publication de la demande: **15.11.2023**
(21) Numéro de dépôt: 23172392.5
(22) Date de dépôt: 09.05.2023
(51) Int. Cl.: A61K 36/185, A61P 17/02, A61K 8/9789, A61Q 19/00

(54) **EXTRAIT DE CISTUS MONSPELIENSIS ET COMPOSITIONS LE COMPRENANT POUR FAVORISER LA CICATRISATION ET LA RÉPARATION DES LÉSIONS CUTANÉES**

(30) Priorité: 09.05.2022 FR 2204371
(71) Demandeur: Pierre Fabre Dermo-Cosmétique, 81500 Lavaur (FR)
(72) Inventeur: LETI, Mathieu, 81106 CASTRES (FR); BIANCHI, Pascale, 81106 CASTRES (FR)
(74) Mandataire: Regimbeau

(57) **Abrégé**

La présente invention concerne un extrait de *Cistus monspeliensis* obtenu par extraction par CO₂ supercritique, plus particulièrement un extrait de parties aériennes fleuries de *Cistus monspeliensis,* pour son utilisation dans le traitement des lésions cutanées.

## Description

### DOMAINE TECHNIQUE DE L'INVENTION

La présente invention concerne un extrait de *Cistus monspeliensis* ou des compositions dermo-cosmétique ou dermatologique comprenant un tel extrait, pour leur utilisation dans la réparation cutanée et la cicatrisation des lésions cutanées.

### ETAT DE LA TECHNIQUE

*Cistus monspeliensis* L. est un arbuste du pourtour méditerranéen communément appelé « Ciste de Montpellier » ou « Montpellier Rockrose ». Il colonise en France les terrains secs, les maquis et les garrigues des côtes méditerranéennes et de Corse. La plante, glutineuse, sécrète un exsudat collant et aromatique. Ses rameaux sont dressés et portent des feuilles persistantes, rugueuses, sessiles ou subsessiles, linéaires-lancéolées ou étroitement elliptiques, trinervées, à marges révolutées. Les inflorescences sont en cymes scorpioïdes unilatérales composées de fleurs de 2 à 3 cm de diamètre, présentant 5 pétales blancs, jamais maculés de pourpre. Les fruits sont des capsules arrondies, facilement écrasées par pression des doigts (Source : TISON J.-M. 2014, Flora Gallica - Flore de France. Biotope Editions, 1195).

Le Ciste de Montpellier fait partie des plantes dites « pionnières ». Sa capacité d'adaptation et sa résistance aux sols pauvres, au sel, ainsi qu'à la sécheresse, lui permettent de coloniser les terrains dégradés des zones côtières méditerranéennes, et de freiner leur érosion. Il s'installe souvent dans les anciennes terres cultivées ou les pâturages à l'abandon.

Plusieurs usages traditionnels de *Cistus monspeliensis* L. sont référencés dans le pourtour méditerranéen, notamment par voie topique. Au Maroc, les feuilles sont utilisées comme antidiarrhéiques et pour ses propriétés anti-inflammatoires. L'usage du Ciste de Montpellier comme infusion, comme épice ou en cas d'asthme est reporté (Demetzos et al., 2001, Planta Medica 67 : 614-618). On trouve également des extraits de *Cistus monspeliensis* L. dans plusieurs références cosmétiques anti-âges.

Les parties aériennes de *Cistus monspeliensis* L. contiennent des diterpènes de type labdane parmi lesquels on peut citer l'acide 8-hydroxylabdan-15-oique ou l'oxyde de manoyle.

Des diterpènes de type clerodane sont également documentés comme l'acide 15-acetoxy-cis-clerodan-3-ene-18-oique (Papaefthimiou et al., 2014, Frontiers in Chemistry 2(35) : 1-19).

*Cistus monspeliensis* L. renferme également plusieurs familles de polyphénols parmi lesquels des flavanoïdes, des tanins, en particulier des tanins ellagiques, et des acides phénoliques (Santagati et al., 2008, Journal of Chromatographie Science 46(2) : 150-156).

Les parties aériennes de *Cistus monspeliensis* L. sont par ailleurs source d'huile essentielle dont les constituants majeurs sont de type terpénique et phénolique (Loizzo et al, 2013, Food and Chemical Toxicology 59 :586-594).

Différents extraits de *Cistus monspeliensis* L. ont fait l'objet d'études pharmacologiques *in vitro* et *in vivo.* Les propriétés antimicrobiennes et antifongiques sont documentées (Bouamama et al., 2006, Journal of Ethnopharmacology 104 : 104-107) comme l'activité anti-Leishmania portée par les diterpènes du Ciste de Montpellier (Fokialakis et al., 2006, Biological and Pharmaceutical Bulletin 29(8) : 1775-1778).

L'activité antioxydante d'un extrait méthanolique de *Cistus monspeliensis* L. a été mise en évidence *in tubo* avec notamment une Cl50 de 3 µg/ml sur un test DPPH (2,2-diphényl 1-picrylhydrazyle). L'activité antioxydante d'un extrait aqueux, son activité protectrice vis-à-vis du clivage de l'ADN, et son activité inhibitrice de la peroxydation lipidique ont également été montrées. Par ailleurs, l'activité antioxydante de l'huile essentielle de *Cistus monspeliensis* L. a également été documentée dans le cadre d'une étude visant à évaluer son intérêt dans la prévention de maladies neurodégénératives (Loizzo et al., 2013).

Il a également été mis en évidence *in vitro* une action inhibitrice des enzymes alphaglucosidase et alpha-amylase d'extraits aqueux et hydro-méthanolique de *Cistus monspeliensis* L., liant la plante à une potentielle activité hypoglycémiante (Sayah et al., 2017, BioMed Research International, 2789482 : 1-7).

Il a été rapporté, que des extraits hexaniques et aqueux de *Cistus monspeliensis* L. sont dotés d'une activité antiproliférative sur différentes lignées cellulaires (Angelopoulou et al., 2001, Planta Medica, 67(2) : 168-202), une activité myorelaxante *ex vivo* a été mise en évidence avec un extrait aqueux de *Cistus monspeliensis* L. (Attaguile et al., 2004, Journal of Ethnopharmacology, 92 : 245-250). L'huile essentielle de *Cistus monspeliensis* L. acitiverait SIRT1 ce qui pourrait suggérer une activité anti-sénescence (Ledrhem et., 2022, Molecules, 27(7), 2053).

Une activité antiinflammatoire d'extraits aqueux et éthanolique de feuilles de *Cistus monspeliensis* L. a été révélée sur différents marqueurs précoces et génériques de l'inflammation sur des tests *in vitro* sur des cellules humaines (Taila et al., 2008, Journal of Pharmacie and Pharmacology, Suppl 1 : A-62, 158).

Les propriétés antiinflammatoires et analgésiques d'un extrait aqueux de partie aérienne de *Cistus monspeliensis* L. ont été mises en évidence *in vivo* par administration par voie orale à des souris et des rats (Sayah et al., 2017, South African Journal of Botany, 113 :160-163). Il est intéressant de noter que l'activité analgésique est obtenue après administration orale sur des modèles de douleur animaux conventionnels comme le test de contorsion par injection d'acide acétique.

Des extraits de *Cistus monspeliensis* L. ont fait l'objet de dépôts de brevets pour une utilisation cosmétique, notamment dans le domaine de l'antiâge. Le brevet FR2819718 cite l'extrait de *Cistus monspeliensis* parmi des extraits lipidiques destinés à un usage cosmétique pour lutter le contre le vieillissement de la peau. Le brevet FR2868307 divulgue un extrait de *Cistus monspeliensis* dans le traitement des rides associées aux contractions musculaires. Le brevet JP2011162504 cite qu'un extrait de *Cistus monspeliensis* induit une production d'ATP. La demande de brevet GB2443388 décrit une association d'algine hydrolysée avec au moins un extrait de *Lavandula stoechas, Helichrysum italicum* et *Cistus monspeliensis* obtenu par hydrodistillation sous vide assistée par micro-ondes dans le traitement de l'acné par voie topique.

Enfin, la demande de brevet WO 2020128223 concerne de nouvelles utilisations cosmétiques et dermatologiques d'un extrait de *Cistus monspeliensis* dans le maintien ou le renforcement de la fonction barrière via notamment le maintien ou l'augmentation de la différenciation cellulaire. Les effets divulgués dans cette demande de brevet ne concernent que la peau saine.

Ces documents de l'art antérieur ne divulguent pas qu'un extrait de *Cistus monspeliensis* L. à fortiori pas un extrait des parties aériennes fleuries de cette plante, présente une activité dans la réparation cutanée et le traitement des lésions cutanées.

La peau est constituée de différents tissus formant une barrière vitale pour l'organisme vis-à-vis de l'environnement extérieur. Cette barrière protège l'organisme contre les agressions extérieures, notamment chimiques ou mécaniques. La peau est constituée de trois parties principales, l'une superficielle, l'épiderme, la partie interne, le derme et une couche plus profonde, l'hypoderme, qui interagissent. L'épiderme humain se compose de quatre à cinq couches distinctes (selon le site anatomique) et de quatre types de cellules qui sont les kératinocytes, très majoritaires, les mélanocytes, les cellules de Langherans et les cellules de Merkel. Chacun de ces types cellulaires contribue par ses fonctions propres au rôle essentiel joué dans l'organisme par la peau, notamment le rôle de protection de l'organisme des agressions extérieures. Cette propriété est appelée fonction barrière. Les cellules épidermiques prolifèrent au niveau de la couche la plus profonde, la couche basale, et se différencient au cours de leur migration vers les couches supérieures pour former successivement la couche épineuse, la couche granuleuse et enfin la couche cornée (ou *stratum corneum*) qui est la couche la plus superficielle de l'épiderme.

La cicatrisation est un ensemble de phénomènes locaux de défense, survenant après une agression : blessure, brûlure, acte dermatologique, intervention chirurgicale. La cicatrisation des plaies est un processus biologique complexe et dynamique qui met en jeu l'interaction de nombreux facteurs locaux et systémiques dans la réparation normale des tissus.

La progression de la cicatrisation comporte plusieurs phases interdépendantes : l'hémostase et l'inflammation, la prolifération et le remodelage. La prolifération implique trois processus bien observables : la granulation, la contraction et la réépithélialisation. Au cours de la granulation, on observe la prolifération et la migration vers le lit de la plaie des cellules qui interviendront dans le reste du processus de réparation. Ainsi, on y retrouve des macrophages, des fibroblastes et des cellules endothéliales. Les macrophages libèrent constamment des facteurs chimiotactiques et des facteurs de croissance. Les fibroblastes construisent la nouvelle matrice cellulaire nécessaire à la croissance des cellules au fond de la plaie. Cet échafaudage favorise la migration cellulaire. Enfin, les cellules endothéliales déclenchent la formation de bourgeons vasculaires qui constitueront de nouveaux capillaires, ce qui permettra de rétablir la perfusion et d'assurer l'apport en oxygène et en nutriments essentiels à l'activité métabolique des cellules dans la plaie. La contraction de la plaie est un mécanisme de réduction de la taille de la plaie et les fibroblastes jouent un rôle de premier plan dans cette contraction.

La ré-épithélialisation consiste en la régénération d'un épiderme qui recouvre une plaie pour reformer une barrière efficace contre l'environnement extérieur, capable de se pigmenter et de retrouver ses fonctions sensorielles et immunitaires. Elle implique donc les processus cellulaires de migration et de prolifération des kératinocytes, et la restauration d'une membrane basale qui recouvre le derme et l'épiderme. Lorsque la migration des cellules basales en direction du centre de la plaie permet aux deux bords de la plaie de se rejoindre, une vague de mitose cellulaire se produit pour combler les espaces laissés par la migration et fournir des cellules pour le tissu épithélial en régénération tridimensionnelle.

Les étapes de prolifération des kératinocytes, des fibroblastes ou des cellules endothéliales peuvent être considérées comme un des phénomènes fonctionnels témoignant de l'activité cicatrisante d'un actif.

Une augmentation de la prolifération des fibroblastes participerait à la cicatrisation d'une plaie profonde (atteinte du derme), alors que l'augmentation de la prolifération et/ou de la migration des kératinocytes participerait à la ré-épithélialisation.

La cicatrisation pose aussi des problèmes cosmétiques à partir du moment où des défauts de cicatrisation, ou une mauvaise cicatrisation, peuvent survenir, par exemple avec l'âge. Le processus de cicatrisation et de récupération des lésions cutanées devient ainsi moins performant, ainsi des marques visibles disgracieuses et quasi permanentes pourront subsister.

Toutes les interventions chirurgicales, de médecine esthétique, de dermatologie ainsi que les lésions traumatiques non médicales comme les éraflures, coupures, égratignures, brûlures, mais aussi pathologiques, comme l'acné, impliquent un phénomène de cicatrisation et entrainent des cicatrices, plus ou moins marquées selon la qualité de cette cicatrisation.

Il existe toujours un besoin de proposer de nouvelles compositions pour favoriser et améliorer la cicatrisation des peaux lésées, permettre une réparation plus rapide des lésions cutanées et obtenir des cicatrices les plus esthétiques possibles.

### RESUME DE L'INVENTION

La présente invention porte sur un extrait de *Cistus monspeliensis* obtenu par extraction par CO₂ supercritique et une composition dermo-cosmétique ou dermatologique comprenant un tel extrait et au moins un excipient dermo-cosmétiquement ou dermatologiquement acceptable pour leur utilisation dans le traitement des lésions cutanées.

D'autres aspects de l'invention sont tels que décrits ci-dessous et dans les revendications.

### DEFINITIONS

Dans la présente description, la plante *Cistus monspeliensis* L. pourra être désignée de manière abrégée par le terme *Cistus monspeliensis.*

Par « extrait de *Cistus monspeliensis* », on entend désigner dans la présente invention, le produit d'extraction de tout ou partie de la plante *Cistus monspeliensis.*

Par « produit d'extraction », on entend au sens de la présente invention, le produit obtenu après extraction de la plante ou d'une partie de la plante, avec un solvant appelé solvant d'extraction, c'est-à-dire un produit présent dans le solvant d'extraction qui peut ensuite être éventuellement sous une forme concentrée ou sèche après évaporation partielle ou totale du solvant d'extraction. Il peut s'agir d'un extrait sec.

Par « extrait sec », on entend au sens de la présente invention, un extrait dépourvu de solvant d'extraction ou de support, ou en contenant uniquement à l'état de traces non significatives. Un tel extrait sec contient ainsi uniquement de la matière issue de *Cistus monspeliensis.* Il peut contenir également des traces non significatives de solvant d'extraction.

Par « environ », on entend au sens de la présente invention, que la valeur concernée peut être inférieure ou supérieure de 10%, notamment de 5%, en particulier de 2%, plus particulièrement de 1% à la valeur indiquée.

Par « solvant moyennement polaire », on entend au sens de la présente invention, un solvant choisi dans le groupe constitué notamment des alcools en C1 à C5, des glycols comme le propylène glycol, le butylène glycol, le butanediol, ou le pentylène glycol, de glycérol, d'acétone, d'esters d'alkyles tels qu'acétate d'éthyle, acétate d'isopropyle de triéthyl citrate, de solvants miscibles à l'eau (un mélange hydro-alcoolique ou un mélange acétone/eau par exemple). Figurent également dans ce groupe des solvants alternatifs de type hydrotropes (molécules amphiphiles solubles dans l'eau et qui, à partir d'une concentration suffisante, peuvent extraire des composés moyennement polaires tels que décrits dans la caractérisation de l'extrait).

Par « solvant apolaire », il faut comprendre au sens de la présente invention, un solvant choisi par exemple parmi l'heptane, l'hexane, le limonène, l'oléate d'éthyle, les hydrocarbures halogénés (par exemple les hydrocarbures chlorés en C1 à C3 tels que le chloroforme ou le dichlorométhane), le CO₂ supercritique, un mélange CO₂ supercritique et éthanol et les mélanges de ces solvants. On peut citer également les solvants 100% biosourcés comme EcoXtract LIPOCOS (Fournisseur Pennakem Europe).

Par « extraction par CO₂ supercritique », on entend au sens de la présente invention, une extraction par du CO₂ dans un état supercritique, c'est-à-dire soumis à une pression et une température supérieures à son point critique. La température doit donc être supérieure à 31°C et la pression supérieure à 74 bars. Dans sa phase supercritique, le CO₂ est un solvant totalement neutre, non toxique, non polluant, non inflammable permettant l'extraction de composés lipophiles. Le CO₂ supercritique peut voir sa polarité modifiée par l'ajout d'un co-solvant polaire comme l'éthanol afin d'élargir la gamme de polarité des molécules extraites. L'extraction par CO₂ supercritique constitue ainsi une alternative privilégiée aux solvants pétrochimiques tels que l'hexane ou l'heptane.

Par « diterpène », on entend au sens de la présente invention des composés en C20 issus du métabolisme du 2^{E}, 6^{E}, 10^{E}-geranyldiphosphate. La structure des diterpènes est très variable, et dépendante de leur biogénèse.

Par « diterpène de type labdane », on entend au sens de la présente invention une classe de diterpènes présentant un squelette bicyclique auquel est rattaché une chaîne à 6 atomes de carbone (cyclique ou acyclique) qui peut ou non contenir un atome d'oxygène.

Par « application topique », on entend au sens de la présente invention une application sur la peau (dont le cuir chevelu), les muqueuses et/ou les cheveux.

Par « dermo-cosmétiquement acceptable » ou « dermatologiquement acceptable », on entend au sens de la présente invention ce qui est utile dans la préparation d'une composition dermo-cosmétique ou dermatologique, qui est généralement sûr, non toxique et ni biologiquement ni autrement non souhaitable et qui est acceptable pour une utilisation dermo-cosmétique ou dermatologique.

### DESCRIPTION DETAILLEE DE L'INVENTION

De façon surprenante et inattendue, les inventeurs ont mis en évidence qu'un extrait de *Cistus monspeliensis* obtenu par extraction par CO₂ supercritique plus particulièrement un extrait de parties aériennes fleuries de *Cistus monspeliensis,* possède une activité biologique sur la peau, basée sur des propriétés de réparation à travers son action sur la migration des kératinocytes. Cette activité est particulièrement intéressante dans la régénération ou réparation tissulaire et la cicatrisation des lésions cutanées.

Ainsi, l'invention concerne un extrait de *Cistus monspeliensis* obtenu par extraction par CO₂ supercritique, plus particulièrement un extrait de parties aériennes fleuries de *Cistus monspeliensis,* pour son utilisation dans le traitement des lésions cutanées. En particulier, le traitement des lésions cutanées comprend l'amélioration de la cicatrisation, la régénération ou la réparation des lésions cutanées.

L'extrait est typiquement sous une forme adaptée à une utilisation par voie topique. L'invention concerne alors un extrait de *Cistus monspeliensis* pour son utilisation selon l'invention, par voie topique.

L'extrait de *Cistus monspeliensis* utile dans le cadre de la présente invention peut être tel que défini ci-après.

### Extrait de Cistus monspeliensis

Dans le cadre de la présente invention, l'extrait de *Cistus monspeliensis* est obtenu à partir d'une ou plusieurs parties de la plante *Cistus monspeliensis* choisie(s) parmi la plante entière, les racines, les parties aériennes, le feuilles, les trichomes, les fruits, les fleurs, et/ou les tiges.

De façon avantageuse, l'extrait de *Cistus monspeliensis* est obtenu à partir de parties aériennes au stade végétatif, de parties aériennes fleuries ou de parties aériennes au stade de fructification de la plante *Cistus monspeliensis.*

De façon plus avantageuse, l'extrait de *Cistus monspeliensis* est obtenu à partir de parties aériennes fleuries de la plante *Cistus monspeliensis.*

La plante ou partie de plante *Cistus monspeliensis* peut être fraiche ou sèche, entière, coupée ou broyée puis soumise à une étape d'extraction. De façon avantageuse, la plante ou partie de plante *Cistus monspeliensis* est sèche et broyée avant d'être soumise à une étape d'extraction.

La plante sèche broyée peut être mouillée dans de l'eau avant extraction.

Dans certains modes de réalisation, l'extrait de *Cistus monspeliensis* est obtenu à partir d'une culture de cellules de *Cistus monspeliensis.*

De préférence, l'extrait de *Cistus monspeliensis* est un extrait obtenu par extraction par CO₂ supercritique, en particulier un extrait obtenu par extraction par CO₂ supercritique avec ou sans ajout d'éthanol comme co-solvant, de parties aériennes fleuries de la plante *Cistus monspeliensis.*

De préférence, l'extrait de *Cistus monspeliensis,* est susceptible d'être obtenu par un procédé tel que décrit ci-après.

Un procédé de préparation d'un extrait de *Cistus monspeliensis* utile dans le cadre de l'invention, comprend une étape d'extraction de tout ou partie de plante *Cistus monspeliensis,* en particulier des parties aériennes fleuries de la plante *Cistus monspeliensis,* par du CO₂ supercritique avec ou sans co-solvant éthanol.

De façon plus avantageuse, il s'agira de CO₂ supercritique avec co-solvant éthanol.

De façon encore plus avantageuse, le mélange CO₂ supercritique/éthanol sera caractérisé par une proportion en CO₂ supercritique/éthanol de 70 : 30 à 99 : 1 (m/m).

Et de façon encore plus avantageuse, le mélange CO₂ supercritique/éthanol est un mélange CO₂ supercritique/éthanol dans la proportion 92 : 8 (m/m).

L'extraction par CO₂ supercritique peut être réalisée à une température comprise entre 32 et 55°C, à une pression comprise entre 200 et 600 bars. Le ratio poids de plante/volume de CO₂ supercritique pouvant varier de 1/10 à 1/100, pendant une durée de 1 minute à 48 heures. L'extraction peut être renouvelée 2 à 3 fois.

A la fin de l'extraction, la plante peut être séchée au moyen de CO₂ supercritique.

L'extrait obtenu est ensuite typiquement filtré afin de récupérer une phase liquide limpide exempte de particules. La phase liquide obtenue peut être plus ou moins concentrée, pouvant aller jusqu'à l'obtention d'un extrait sec.

Un support peut être ajouté lors de l'étape de concentration de façon à obtenir un extrait contenant de 1 à 80% en poids d'extrait sec.

Le support peut être de la maltodextrine, du lactose, de la silice, de la glycérine, un glycol, une huile végétale, du triethyl citrate, de l'oléate d'éthyle, du dicaprylyl carbonate, de l'octyldodécanol, un hydrotrope, un mélange eau/solubilisant ou eau/tensioactif, ou tout autre support cosmétologiquement acceptable et solubilisant l'extrait, préférentiellement d'origine biosourcée comme par exemple des glycols biosourcés (1,2-pentanediol ; 1,3-butanediol ; 1,3-propanediol...), des acides gras estérifiés et également les hydrotropes comme par exemple les alkyls glycosides (Sepiclear, Apyclean, APXC4...).

Dans certains modes de réalisation, l'extrait de *Cistus monspeliensis* peut être décoloré, par exemple sur charbon actif.

### Utilisations de l'extrait de Cistus monspeliensis

L'extrait de *Cistus monspeliensis* tel que décrit ci-dessus peut être utilisé dans le traitement des lésions cutanées.

Il a été mis en évidence que l'extrait de *Cistus monspeliensis* favorise la migration des kératinocytes. L'invention concerne également un extrait de *Cistus monspeliensis,* pour son utilisation afin de favoriser la migration des kératinocytes.

En particulier, le traitement des lésions cutanées comprend l'amélioration de la cicatrisation, la régénération ou la réparation des lésions cutanées.

Les lésions cutanées peuvent être choisies parmi les lésions post-traumatiques, les lésions chirurgicales ou post-chirurgicales, les lésions postérieures à un acte de dermatologie et les lésions postérieures à un acte de médecine esthétique.

Plus spécifiquement, l'invention concerne un extrait de *Cistus monspeliensis,* pour son utilisation pour améliorer la cicatrisation des lésions cutanées, préférentiellement les lésions cutanées sont choisies dans le groupe consistant en les lésions post-traumatiques, les lésions post-chirurgicales, les lésions postérieures à un acte de dermatologie, et les lésions postérieures à un acte de médecine esthétique.

Les lésions cutanées post-traumatiques peuvent être choisies parmi les écorchures, les brûlures, les coups de soleil, les égratignures, les coupures et les éraflures.

Les lésions cutanées chirurgicales ou post-chirurgicales peuvent être choisies parmi les lésions postérieures et consécutives à une biopsie, à une exérèse et à une suture par fil, par bandelettes, par agrafes ou par colle.

Les lésions postérieures à un acte de dermatologie peuvent être choisies parmi les lésions postérieures et consécutives à une biopsie, à une exérèse, à un traitement par laser, à une cryothérapie.

Les lésions postérieures à un acte de médecine esthétique peuvent être choisies parmi les lésions postérieures et consécutives à un peeling, à une injection sous-cutanée, à une pose de fil tenseur, à un traitement par lumière LED (diode électroluminescente), à un traitement de dermabrasion, à un traitement d'éclaircissement, à un traitement laser ou à une carboxythérapie.

### Compositions dermo-cosmétique ou dermatologique et leurs utilisations

La présente invention concerne une composition dermo-cosmétique ou dermatologique comprenant au moins un extrait de *Cistus monspeliensis* tel que décrit ci-avant, avec au moins un excipient dermo-cosmétiquement ou dermatologiquement acceptable, pour son utilisation dans le traitement des lésions cutanées.

La composition est typiquement sous une forme adaptée à une utilisation par voie topique. Les compositions dermo-cosmétiques ou dermatologiques peuvent se présenter sous les formes qui sont habituellement connues pour une administration topique, c'est-à-dire notamment les lotions, les laits, les émulsions, les sérums, les baumes, les masques, les crèmes, les dispersions, les gels, les mousses, les sprays, les shampoings.

Les compositions dermo-cosmétique ou dermatologique, outre l'extrait de *Cistus monspeliensis* et un excipient dermo-cosmétiquement ou dermatologiquement acceptable, peuvent également contenir des agents tensioactifs, des agents complexants, des conservateurs, des antioxydants comme les tocophérols, des agents stabilisants, des émulsifiants, des épaississants, des gélifiants, des humectants, des émollients, des oligo-éléments, des huiles essentielles, des parfums, des colorants, des agents matifiants, des filtres chimiques ou minéraux, des agents hydratants, des eaux thermales etc.

Selon un mode particulier de réalisation, l'invention concerne une composition dermo-cosmétique ou dermatologique comprenant au moins un extrait de *Cistus monspeliensis* avec au moins un excipient dermo-cosmétiquement ou dermatologiquement acceptable pour son utilisation afin de favoriser la migration des kératinocytes.

Le traitement des lésions cutanées comprend en particulier l'amélioration de la cicatrisation, la régénération ou la réparation des lésions cutanées.

Ainsi, l'invention concerne plus particulièrement une composition dermo-cosmétique ou dermatologique comprenant au moins un extrait de *Cistus monspeliensis* avec au moins un excipient dermo-cosmétiquement ou dermatologiquement acceptable pour son utilisation pour améliorer la régénération, la cicatrisation ou la réparation des lésions cutanées.

Les lésions cutanées peuvent être choisies parmi les lésions post-traumatiques, les lésions chirurgicales ou post-chirurgicales, les lésions postérieures à un acte de dermatologie et les lésions postérieures à un acte de médecine esthétique.

Les lésions cutanées sont préférentiellement choisies dans le groupe consistant en les lésions post-traumatiques, les lésions post-chirurgicales, les lésions postérieures à un acte de dermatologie et les lésions postérieures à un acte de médecine esthétique.

De manière particulière, les lésions post-traumatiques sont choisies parmi les écorchures, les brûlures, les coups de soleil, les égratignures, les coupures et les éraflures.

Les lésions chirurgicales ou post-chirurgicales sont choisies parmi les lésions postérieures et consécutives à une biopsie, à une exérèse ou à une suture par fil, par bandelettes, par agrafes ou par colle.

Les lésions postérieures à un acte de dermatologie peuvent être choisies parmi les lésions postérieures et consécutives à une biopsie, à une exérèse, à un traitement par laser et à une cryothérapie.

Les lésions postérieures à un acte de médecine esthétique peuvent être choisies parmi les lésions postérieures et consécutives à un peeling, à une injection sous-cutanée, à une pose de fil tenseur, à un traitement par lumière LED, à un traitement de dermabrasion, à un traitement d'éclaircissement, à un traitement laser ou une carboxythérapie.

De manière préférée, les lésions cutanées sont choisies dans le groupe consistant en les écorchures, les brûlures, les coups de soleil, les égratignures, les éraflures et les sutures.

La composition dermo-cosmétique ou dermatologique, comprend typiquement de 0,01 à 5% en poids, de préférence 0,01 à 4% en poids, de manière préférée de 0,02 à 2% en poids, de manière encore préférée de 0,02 à 1,5% d'extrait de *Cistus monspeliensis,* en poids d'extrait sec de *Cistus monspeliensis* par rapport au poids total de la composition. Ce pourcentage en poids d'extrait sec s'entend hors poids de l'éventuel support de séchage, s'il y en a, et ne concerne que l'extrait sec de plante.

La présente invention concerne une méthode cosmétique d'atténuation des défauts de cicatrisation cutanée comprenant l'application topique d'une quantité efficace d'un extrait de *Cistus monspeliensis* tel que défini ci-avant, ou d'une composition dermo-cosmétique ou dermatologique, comprenant au moins un extrait de *Cistus monspeliensis* tel que décrit ci-avant avec au moins un excipient dermo-cosmétiquement ou dermatologiquement acceptable et formulée sous une forme adaptée pour une application topique.

Selon un mode particulier de réalisation, l'invention vise l'utilisation, non thérapeutique, d'une composition telle que définie ci-dessus pour l'atténuation des défauts esthétiques de cicatrisation cutanée.

Les défauts de cicatrisation cutanée peuvent être consécutifs à des lésions post-traumatiques, à des lésions chirurgicales ou post-chirurgicales, à des lésions postérieures à un acte de dermatologie ou à des lésions postérieures à un acte de médecine esthétique. Les lésions post-traumatiques sont choisies parmi les écorchures, les brûlures, les coups de soleil, les égratignures, les coupures, les éraflures. Les lésions chirurgicales ou post-chirurgicales sont choisies parmi les lésions postérieures et consécutives à une biopsie, à une exérèse, à une suture par fil, par bandelettes, par agrafes, ou par colle. Les lésions postérieures à un acte dermatologie peuvent être choisies parmi les lésions postérieures et consécutives à une biopsie, à une exérèse, à un traitement par laser, à une cryothérapie. Les lésions postérieures à un acte de médecine esthétique peuvent être choisies parmi les lésions postérieures et consécutives à un peeling, à une injection sous-cutanée, à une pose de fil tenseur, à un traitement par lumière LED, à un traitement de dermabrasion, à un traitement d'éclaircissement, à un traitement laser ou une carboxythérapie.

La présente invention vise encore l'utilisation d'un extrait de *Cistus monspeliensis* tel que défini ci-avant pour la fabrication d'une composition dermo-cosmétique destinée à une application topique sur des cicatrices.

Enfin, l'invention vise encore un procédé de fabrication d'une composition dermo-cosmétique destinée à une application topique sur des cicatrices comprenant le mélange d'un extrait de *Cistus monspeliensis* tel que défini ci-avant avec au moins un excipient dermo-cosmétiquement acceptable.

Les exemples qui suivent illustrent l'invention sans en limiter la portée.

### EXEMPLES

### Exemple 1 : extraction par CO₂ supercritique

300 grammes de parties aériennes fleuries sèches broyées de *Cistus monspeliensis* sont extraits par CO₂ supercritique à une pression de 250 bars et une température de 40°C à un débit de 10 kg/h pendant 180 minutes. Après détente jusqu'à pression atmosphérique et retour à température ambiante, l'installation est rincée à l'éthanol pour récupérer la totalité de l'extrait. L'extrait est filtré sur plaque de filtration K900 et l'éthanol est évaporé de manière à obtenir 34 grammes d'une cire verdâtre avec un rendement massique de 11%.

### Exemple 2 : extraction par CO₂ supercritique avec co-solvant éthanol

300 grammes de parties aériennes fleuries sèches broyées de *Cistus monspeliensis* sont extraits par CO₂ supercritique avec co-solvant éthanol 96° à une pression de 200 bars et une température de 40°C à un débit de 10 kg/h en CO₂ et 0,8 kg/h en éthanol 96° pendant 180 minutes. Après détente jusqu'à pression atmosphérique et retour à température ambiante, l'extrait est filtré sur plaque de filtration K900 et l'éthanol est évaporé de manière à obtenir 35 grammes d'une cire verdâtre avec un rendement massique de 12%.

### Exemple 3 : extraction par CO₂ supercritique avec co-solvant éthanol et décoloration au charbon actif

5 kilogrammes de parties aériennes fleuries sèches broyées de *Cistus monspeliensis* sont extraits par CO₂ supercritique avec co-solvant éthanol 96° à une pression de 200 bars et une température de 40°C à un débit de 10 kg/h en CO₂ et 0,8 kg/h en éthanol 96° pendant 180 minutes. Après détente jusqu'à pression atmosphérique et retour à température ambiante, l'extrait est filtré sur plaque de filtration K900 puis décoloré au charbon actif (0,2% p/v de solution). Après nouvelle filtration sur K900, l'éthanol est évaporé de manière à obtenir 524 grammes d'une cire orangée avec un rendement massique de 10%.

### Exemple 4 : extraction par CO₂ supercritique avec co-solvant éthanol et décoloration au charbon actif et mise sur support fluide

10 kilogrammes de parties aériennes fleuries sèches broyées de *Cistus monspeliensis* sont extraits par CO₂ supercritique avec co-solvant éthanol 96° à une pression de 200 bars et une température de 47°C à un débit de 40 kg/h en CO₂ et 3,2 kg/h en éthanol 96° pendant 7 heures. Après détente jusqu'à pression atmosphérique et retour à température ambiante, l'extrait est filtré sur plaque de filtration K900 puis décoloré au charbon actif (0,5% p/v de solution). Après nouvelle filtration sur K900, l'extrait est séché sur 1,2-pentanediol de manière à obtenir 5 kg d'extrait sous forme d'un liquide visqueux orangé.

### Exemple 5 : évaluation d'un extrait de parties aériennes fleuries de Cistus monspeliensis sur la migration des kératinocytes

Cette activité de migration est particulièrement intéressante dans la régénération tissulaire et la cicatrisation des lésions cutanées. En effet, la migration des cellules épithéliales est une étape importante du développement et du processus de réparation tissulaire, tels que l'embryogénèse et la cicatrisation. Les mécanismes d'initiation, de coordination et d'arrêt des mouvements des cellules, ne sont pas complètement élucidés, cependant le rôle primordial de la migration cellulaire est bien établi. Plusieurs agents susceptibles de stimuler ce processus cellulaire ont été caractérisés, telles que certaines protéines matricielles ou cytoplasmiques (Santoro and Gaudino, Exp. Cell Res. 304(1), 274-286, 2005). Lors de la cicatrisation cutanée et dans les affections inflammatoires chroniques dermatologiques, les kératinocytes sont « activés » pour entreprendre le processus de migration. Les cellules voient alors leur phénotype influencé par les interactions avec la matrice extracellulaire d'une part et par les interactions cellules-cellules, d'autre part (McMillan et al., J. Derm. Sci. 31(3), 169-177, 2003). Les kératinocytes de l'assise basale des berges d'une plaie, migrent sur la plaie et la recouvrent. En effet, les kératinocytes sont activés au contact de la fibronectine, du collagène dermique interstitiel (type I), du collagène IV et de la laminine 5 de la lame basale. Ils sont aussi régulés par certains facteurs de croissance polypeptidiques comme le TGFβ, TGFa et l'EGF. De plus des cytokines (IL-1, TNFa) et chémokines (RANTES et IL-8) contribuent aussi à augmenter la vitesse de ré-épithélialisation d'une plaie, à la suite de l'activation kératinocytaire (Szabo et al., J. Invest. Dermatol. 117(5), 1083-1090, 2001).

Le but de cette étude était d'évaluer la capacité d'un extrait de parties aériennes fleuries de *Cistus monspeliensis* pour favoriser la réparation cutanée dans un modèle *in vitro* de cicatrisation épidermique. A cette fin, les inventeurs ont utilisé un dispositif développé par l'équipe de G. Ponzio (Turchi et al., J. Invest. Dermatol. 119, 56-63, 2002). Un scarificateur hélicoïdal permet de réaliser des blessures (ie lésions) de grande taille au sein de tapis cellulaires, et d'analyser le processus de cicatrisation à la fois sur le plan fonctionnel avec une analyse et quantification visuelle de la migration et la prolifération des cellules par des techniques d'immunofluorescence ou de vidéo microscopie à et à la fois moléculaire avec une analyse des variations d'expression des gènes au cours du processus de cicatrisation par des approches globales. Cela est impossible avec les modèles *in vitro* classiquement utilisés dans ce domaine de la cicatrisation dans lequel des lésions mécaniques calibrées sont réalisées dans les cultures des kératinocytes.

L'extrait de parties aériennes fleuries de *Cistus monspeliensis* utilisé dans cette étude a été préparé selon l'exemple 3. Cet extrait a été solubilisé et stocké à 100 mg/ml dans du DMSO (100 mg d'extrait sec préparé selon l'exemple 3, dans 1 ml de DMSO), les concentrations testées sont 5 et 10 µg/ml. Un contrôle positif connu pour stimuler la migration des kératinocytes est également testé dans cette étude, il s'agit de l'EGF (Epidermal Growth Factor) à la concentration de 6 mg/ml, également solubilisé dans le DMSO.

L'étude a été réalisée sur des kératinocytes normaux humains isolés à partir d'exérèses cutanées provenant de chirurgies esthétiques. Les kératinocytes ont été cultivés sur des plaques de 60 mm de diamètre et leur croissance se fait en milieu KSFM (Keratinocyte Serum-Free Growth Medium). Après 48 heures de croissance, les cellules ont été déprivées de compléments pendant 24 heures. Les lésions ont été réalisées en présence ou non (contrôle) des produits à tester selon la méthodologie explicitée plus avant. Six heures après les lésions, les cellules ont été fixées et ensuite colorées à l'hémalun-éosine. Des échantillons servant de contrôle ont également été inclus en fixant les cellules immédiatement après la lésion. Les expériences ont été réalisées sur 3 lots différents de kératinocytes normaux humains.

Pour chaque plaque, six images correspondantes à six zones différentes ont été prises à l'aide d'un microscope EVOS au grossissement X 4. La zone centrale ainsi que les bords de la plaque ont été exclus pour éviter les lésions irrégulières qui pourraient fausser l'analyse. A partir de ces zones, le pourcentage de surface dépourvue de cellules est calculé grâce au logiciel TScratch 1.0 (MATLAB^{®}), on parle de surface ouverte qui correspond à la surface blessée. Pour chaque donneur, le pourcentage de fermeture de la plaie après 6 heures est déterminé comme suit :
Le pourcentage de fermeture des plaies par rapport aux cellules non traitées = % de surface ouverte (non traitée) - % de surface ouverte (d'un groupe traité) / % de surface ouverte (non traitée).

Les résultats de cette étude sont résumés dans le Tableau 1 ci-dessous :

**Tableau 1 : Moyenne % de fermeture des plaies par rapport aux cellules non traitées (contrôle)**

| **Contrôle (blessé sans traitement)** | **Actif de référence EGF (30 mg/ml)** | **Extrait de *Cistus monspeliensis* (5 µg/ml)** | **Extrait de *Cistus monspeliensis* (10 µg/ml)** |
|---|---|---|---|
| 100 | + 207,4 % vs contrôle | + 70,0 % vs contrôle | + 120,2 % vs contrôle |
| - | P<0,001 | NSS | P<0,05 |

| | | | |
|---|---|---|---|
| NSS : non statistiquement significatif : p<0,05 : comparaison inter-groupes (test Anova EliStats macro) | | | |

L'actif de référence augmente de façon significative la migration des kératinocytes, ce résultat était attendu, il valide donc le test. L'extrait de parties aériennes fleuries de *Cistus monspeliensis* dès la concentration de 5 µg/ml tend à augmenter la migration des kératinocytes. A 10 µg/ml l'extrait de *Cistus monspeliensis* stimule très fortement et de manière significative la migration des kératinocytes et la fermeture des plaies 6 heures après la lésion. Il est à noter que le véhicule (DMSO) n'a pas d'effet significatif sur la migration des kératinocytes.

Les inventeurs mettent clairement en évidence, qu'un extrait de parties aériennes fleuries de *Cistus monspeliensis* peut être utile dans la réparation et la cicatrisation cutanée.

## Revendications

1. Extrait de *Cistus monspeliensis* obtenu par extraction par CO₂ supercritique pour son utilisation dans le traitement des lésions cutanées.

2. Extrait de *Cistus monspeliensis* pour son utilisation selon la revendication 1, **caractérisé en ce que** ledit traitement des lésions cutanées comprend l'amélioration de la cicatrisation, la régénération ou la réparation des lésions cutanées.

3. Extrait de *Cistus monspeliensis* pour son utilisation selon l'une quelconque des revendications 1 et 2, **caractérisé en ce que** l'extrait de *Cistus monspeliensis* favorise la migration des kératinocytes.

4. Extrait de *Cistus monspeliensis* pour son utilisation selon l'une des revendications 1 à 3, **caractérisé en ce que** l'extrait de *Cistus monspeliensis* est obtenu à partir des parties aériennes fleuries de la plante.

5. Extrait de *Cistus monspeliensis* pour son utilisation selon l'une des revendications 1 à 4, **caractérisé en ce que** l'extrait de *Cistus monspeliensis* est obtenu par extraction par CO₂ supercritique avec ajout d'éthanol comme co-solvant.

6. Composition dermo-cosmétique ou dermatologique comprenant au moins un extrait de *Cistus monspeliensis,* l'extrait de *Cistus monspeliensis* pouvant être tel que défini dans la revendication 1, 4 ou 5, avec au moins un excipient dermo-cosmétiquement ou dermatologiquement acceptable, pour son utilisation dans le traitement des lésions cutanées.

7. Composition dermo-cosmétique ou dermatologique pour son utilisation selon la revendication 6, **caractérisée en ce que** ledit traitement des lésions cutanées comprend l'amélioration de la cicatrisation, la régénération ou la réparation des lésions cutanées.

8. Composition dermo-cosmétique ou dermatologique pour son utilisation selon l'une quelconque des revendications 6 et 7, **caractérisée en ce que** l'extrait de *Cistus monspeliensis* favorise la migration des kératinocytes.

9. Composition dermo-cosmétique ou dermatologique pour son utilisation selon l'une quelconque des revendications 6 à 8, **caractérisée en ce qu'**elle comprend de 0,01 à 5%, de préférence 0,02 à 2% d'extrait de *Cistus monspeliensis* en poids d'extrait sec par rapport au poids total de la composition.

10. Extrait de *Cistus monspeliensis* pour son utilisation selon l'une quelconque des revendications 1 à 5 ou composition dermo-cosmétique ou dermatologique pour son utilisation selon l'une quelconque des revendications 6 à 9, **caractérisée en ce que** les lésions cutanées sont choisies parmi les lésions post-traumatiques, les lésions chirurgicales ou post-chirurgicales, les lésions postérieures à un acte de dermatologie, et les lésions postérieures à un acte de médecine esthétique.

11. Extrait de *Cistus monspeliensis* pour son utilisation ou composition dermo-cosmétique ou dermatologique pour son utilisation, selon la revendication 10, **caractérisée en ce que** les lésions cutanées post-traumatiques sont choisies dans le groupe consistant en les écorchures, les brûlures, les coups de soleil, les égratignures, les coupures et les éraflures.

12. Extrait de *Cistus monspeliensis* pour son utilisation ou composition dermo-cosmétique ou dermatologique pour son utilisation, selon la revendication 10, **caractérisée en ce que** les lésions cutanées chirurgicales ou post-chirurgicales sont choisies parmi les lésions postérieures et consécutives à une biopsie, à une exérèse, et à une suture par fil, par bandelettes, par agrafes ou par colle.

13. Extrait de *Cistus monspeliensis* pour son utilisation ou composition dermo-cosmétique ou dermatologique pour son utilisation, selon la revendication 10, **caractérisée en ce que** les lésions cutanées postérieures à un acte de dermatologie sont choisies parmi les lésions postérieures et consécutives à une biopsie, à une exérèse, à un traitement par laser, et à une cryothérapie.

14. Extrait de *Cistus monspeliensis* pour son utilisation ou composition dermo-cosmétique ou dermatologique pour son utilisation, selon la revendication 10, **caractérisée en ce que** les lésions cutanées postérieures à un acte de médecine esthétique sont choisies parmi les lésions postérieures et consécutives à un peeling, à une injection sous cutanée, à une pose de fil tenseur, à un traitement par lumière LED, à un traitement de dermabrasion, à un traitement d'éclaircissement, à un traitement laser ou à une carboxythérapie.
